# EUROPEAN PATENT SPECIFICATION

(11) **EP 0 556 618 B1**
(45) Date of publication and mention of the grant of the patent: **29.10.1997**
(21) Application number: 93101376.7
(22) Date of filing: 29.01.1993
(51) Int. Cl.: A61M 25/06

(54) **Surgical dilator**
Chirurgischer Dilator
Dilatateur chirurgical

(30) Priority: 31.01.1992 US 830000
(43) Date of publication of application: 25.08.1993
(73) Proprietor: Becton, Dickinson and Company, Franklin Lakes, New Jersey 07417-1880 (US)
(72) Inventor: Barrett, Joseph H.Jr., Kaysville Utah 84037 (US); Brimhall, Greg L., West Jordan Utah 84084 (US); Orr, Douglas P., Sandy Utah 84070 (US)
(74) Representative: Bosotti, Luciano

(56) References cited:
- WO-A-92/08513
- GB-A- 2 179 258
- US-A- 5 057 083

## Description

### BACKGROUND OF THE INVENTION

### 1. Field of the Invention

This invention relates to the field of medical catheter insertion in general and in particular to dilators for use with such catheters. The invention is directed to a surgical dilator for dilating a vessel prior to the introduction of a catheter into the vessel having the features set forth in the pre-characterising portion of Claim 1. Such a dilator is known, e.g. from US-A-5 057 083.

### 2. Description of the Prior Art

Surgical dilators are used to increase the size of a perforation and to dilate a vessel such as a vein or artery so that a catheter may be inserted into the vessel.

A dilator is, in most cases, a tube having a tapered end for insertion into a vessel. The dilator is formed of a semi-rigid material in order to give it sufficient rigidity during insertion through a puncture site. The known methods of introducing catheters into vessels have meant that known dilators have a connection for the attachment of a syringe having a conventional luer connector which is used in the insertion process. Such dilators are intended to be inserted and then removed almost immediately.

Prior art dilators have couplings such as liner fittings which are useful when they are introduced into the vessel because they allow the dilator to be introduced at the same time as the needle makes the initial puncture. The conventional luer fittings and other couplings such as threaded couplings found on the proximal ends of prior art dilator hubs allow their attachment to syringes and therefore also allow intravenous administration devices to be attached to the dilators. Such conventional luer fittings and other couplings are well known in the art and are referred to collectively as luer connectors.

It is an object of the present invention to provide a dilator to which an intravenous administration device cannot readily be attached.

There are several methods of using a dilator to assist in the introduction of a catheter into a vessel.

One method of using a dilator includes passing a needle through the central bore of the dilator until the sharpened tip of the needle extends distally beyond the distal tip of the dilator. According to this insertion method, the needle tip first punctures the surgical site establishing a hole having a diameter equal to that of the needle. Further insertion causes the dilator to enter the puncture site and in turn the vessel into which a catheter is to be inserted. Since the distal end of the dilator has a generally conical shape, insertion of the dilator further enlarges the puncture site. The entry of the dilator into a vessel into which a catheter is to be inserted also dilates the vessel which facilitates the insertion of the catheter.

The dilator is surrounded by a concentric sheath catheter with a blunt end and made of a relatively soft material. The needle makes the initial puncture in the vessel and the dilator dilates the vessel. Thereafter the sheath catheter is introduced into the vessel and the needle and dilator are removed.

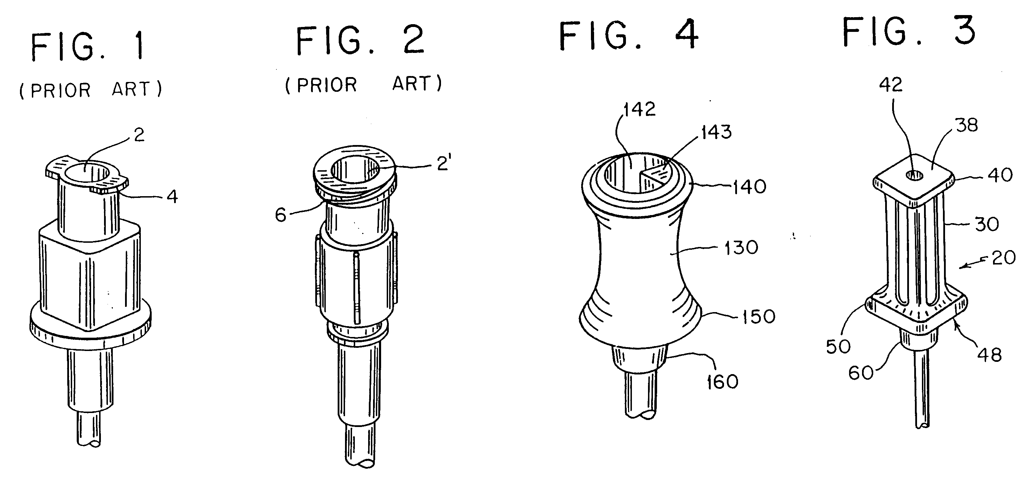

Since the dilators used in this technique are, at least temporarily, coupled with a needle, prior art dilator hubs are designed with a luer connector mechanism. Figures 1 and 2 illustrate such dilator hubs commonly used in the surgical field.

In an alternative insertion method, commonly referred to as the "Seldinger Technique," an introducer needle attached to a syringe is inserted into the desired location and a guide wire is fed through the introducer needle and the puncture site into the vessel. While holding the guide wire in place, the introducer needle is then removed. The proximal end of the guide wire is then fed into the distal end of a dilator which is passed over the guide wire and through the puncture site to enlarge the puncture site and dilate the vessel into which a catheter is to be inserted. The dilator is then removed from the guide wire. A catheter is fed over the guide wire and advanced through the puncture site to the desired location.

In the Seldinger Technique, the dilator may be surrounded by a sheath catheter. In that case, the sheath catheter is slid over the dilator and advanced along the guide wire and into the vessel. The dilator and the guide wire are then removed. Prior art dilators used with the Seldinger Technique, like the dilators used in conventional catheterization procedures, have luer connectors on their proximal ends. The present invention is primarily intended to be used in the Seldinger Technique, although it may also be used in other catheterization techniques.

Another disadvantage inherent in the dilator hubs known in the art is that they do not fit the shape of the user's finger and thumb. See, for example, the dilator hubs shown in prior art Figures 1 and 2.

Another aspect of the present invention is therefore an ergonomically designed dilator hub adapted to fit comfortably and safely between a thumb and a second finger of medical personnel.

The following United States Patents all show prior art dilators having connectors such as luer connectors on their proximal ends: 4,609,370; 4,650,472; 4,772,266; 4,850,975; 4,961,729; 5,011,478.

### SUMMARY OF THE INVENTION

The present invention, having the further features set forth in Claim 1. is a surgical dilator having an improved dilator hub which prevents the attachment to or insertion into the dilator of other surgical devices such as intravenous administration devices, syringes, tubes or the like. Contrary to known devices which include luer connectors, the dilator of the present invention renders the securing of a syringe or intravenous administration device to the proximal end of the hub virtually impossible. As described in further detail below, the present invention comprises a dilator having a proximal opening which departs significantly from the prior art in that it prevents the connection of luer fittings or the like and the insertion of tubes or the like and in that it has an ergonomically designed hub.

### BRIEF DESCRIPTION OF THE DRAWINGS

Figure 1 illustrates a prior art dilator hub having a bayonet-type coupler and a luer fitting.

Figure 2 also discloses a known dilator hub having a luer fitting with a threaded-type coupler.

Figure 3 is a perspective view of a first embodiment of a dilator hub of the present invention.

Figure 4 is a perspective view of an alternative embodiment of the present invention.

Figure 5 is a perspective view of a dilator having a dilator hub as shown in Figure 3.

Figure 6 is a side view of the dilator shown in Figure 5.

Figure 7 is an end view taken from the proximal end of the dilator shown in Figure 6.

Figure 8 is an end view taken from the distal end of the dilator shown in Figure 6.

Figure 9 is a perspective view of a dilator having a dilator hub as illustrated in Figure 4.

Figure 10 is a cross-sectional side view of the dilator shown in Figure 9.

Figure 11 is an end view of the dilator shown in Figure 10 taken from the proximal end.

Figure 12 is an end view of the dilator shown in Figure 10 taken from the distal end.

Figure 13A-D illustrate the use of dilator in the Seldinger Technique.

### DETAILED DESCRIPTION OF THE PREFERRED EMBODIMENT

This invention may be embodied in various forms. The following is a detailed description of a preferred embodiment of the invention. This disclosure is to be considered as exemplary of the principles of the invention and it is not intended to limit the scope of the invention as claimed.

Figures 1 and 2 illustrate dilator hubs commonly used in the medical field. Both of these dilator hubs are provided with luer fittings having openings 2,2' at their proximal ends. A luer fitting is a coupling mechanism for two medical devices, such as a dilator hub and a syringe or catheter, engaged in a friction or interference fit. A luer fitting comprises complementary male and female parts which interconnect. Such fittings are well known in the art. Typical openings adapted to receive luer fittings like the openings 2,2' used in the United States have diameters of about 0.43 cm (0.170") at the end of a passageway having a diameter which decreases to a diameter of about 0.39 cm (0.154") distally of the proximal opening. To further insure a secure coupling between the dilator and another medical device, prior art dilators comprise further coupling mechanisms, such as the coupler having opposing ears 4 shown in FIG. 1. A further additional securing device is shown on the dilator hub of the prior art illustrated in Figure 2 in the form of a threaded connection.

Figures 3 and 5-8 illustrate a first embodiment of the invention. This embodiment of the invention comprises a dilator 10 comprising a hollow tube 11 having a semi-rigid, lumen 15 with a generally conical distal end 12, a distal hole 17 and a dilator hub 20 having a gripping region 30, a proximal end 38, a proximal flange 40, a distal hub end 48 and a distal flange 50 to provide a secure attachment between dilator hub 20 and tube 11. The term gripping region 30 is not intended to include attachment collar 60.

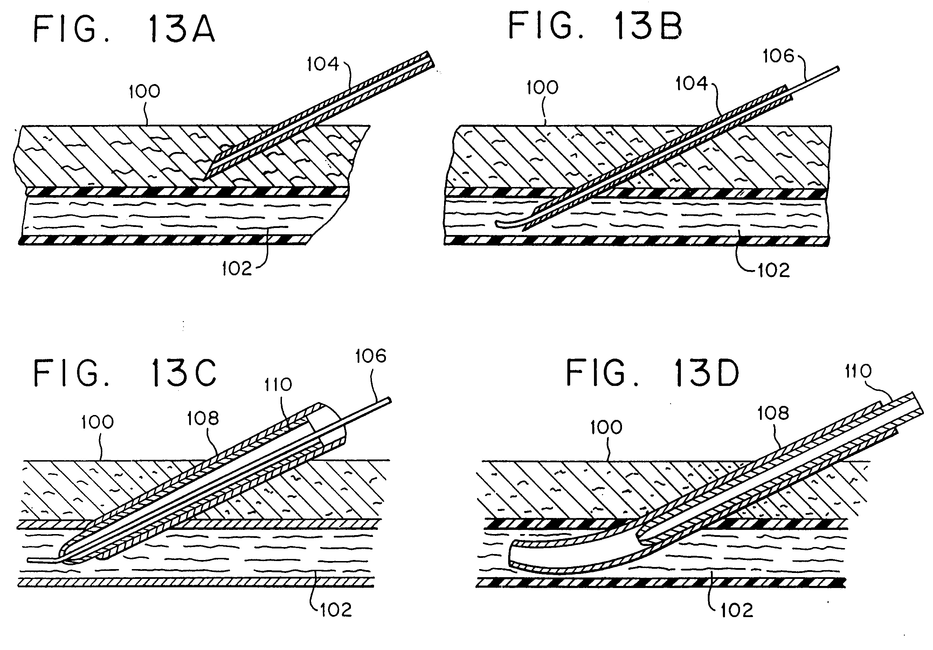

As shown in Figure 3, the proximal end 38, unlike in prior art dilators, is provided with an opening 42 to lumen 15 specifically designed to prevent connection of a luer fitting but to allow entry of a needle and or guide wire 106 (See Figs. 13B and 13C). According to this embodiment of the invention, opening 42 is dimensioned such that a standard male luer fitting will not fit into opening 42 and proximal end 38 may be dimensioned so that a standard female luer fitting will not fit over it or couple with it. Proximal opening 42 should advantageously therefore have a diameter of about 0.05 cm to 0.343 cm (0.020 inches to 0.135) inches, preferably about 0.165 cm to 0.279 cm (0.065 inches to 0.110 inches).

Hub 20 is also shaped so that it is not possible to connect conventional male or female luer fittings to it. In the first embodiment, proximal flange 40 accomplishes this purpose. The shape of hub 20 has the added benefit of providing grip for the dilator which is more ergonomic than the gripping regions of prior art dilators.

Alternative designs will also prevent the engagement of another medical device to the dilator hub and are included in this invention. For example, lumen 15 in the vicinity of proximal end 38 may have an irregular shape or may be provided with an obstruction 143 as shown in the alternative embodiment illustrated in Figs. 3, 9, 10, 11 and 12 which could deny entry to or attachment of a luer fitting. Those skilled in the art will also appreciate that at least when the Seldinger Technique is used, the absence of a generally conically-shaped passageway in the proximal end of dilator hub 20 will not impede the use of the dilator, therefore, as long as a guide wire can pass smoothly through the hollow tube and dilator hub exiting at proximal opening.

The invention may be used in several ways. In the preferred method (the Seldinger Technique) illustrated in Figs. 13A-D, a needle 104, attached to a syringe (not shown) is used to pierce the skin 100 and make an initial opening in a vessel 102 (see Figs. 13A and B). The syringe is then removed and a guide wire 106 is fed through the needle lumen and into the vessel (Fig. 13B). The needle is then removed and the guide wire is left in the vessel 102. At this point a scalpel may be used to enlarge the skin entry site. A dilator 110 having a concentric catheter 108 over it is threaded over the guide wire. The distal end of the dilator 110 protrudes from the catheter sheath 108. The distal end of the dilator 110 is introduced into the hole made by the needle and is fed using a twisting motion into the vessel 102, thus dilating the vessel 102 (Fig. 13C). The catheter 108 is then advanced into the vessel 102. Once the catheter 108 is sufficiently far into the vessel 102, the guide wire 106 and the dilator 110 are removed (Fig. 13D).

In an alternative method, a needle, catheter, dilator combination is used. The needle is sheathed with a catheter which in turn is surrounded by the dilator. The needle pierces the skin and enters the vessel. The vessel is dilated by the dilator and the catheter is fed into the vessel. The dilator and needle are then removed.

Those skilled in the art will appreciate that there are several ways of using the dilator of this invention in combination with a needle or guidewire and a catheter which have substantially the same end result.

Figures 4, 9, 10, 11 and 12 show an alternative embodiment of the invention.

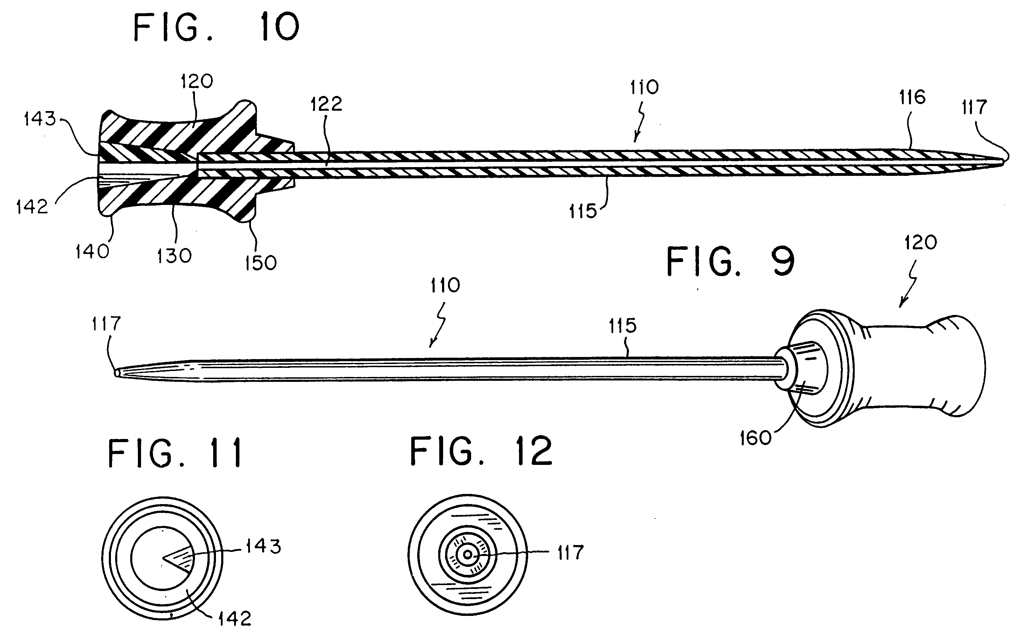

The proximal opening 142 of this embodiment of the present invention does not necessarily have to be smaller than the opening of a conventional luer fitting since proximal lumen 142 is provided with an additional obstruction 143 which prevents the insertion of a luer fitting. According to this alternative embodiment, proximal lumen 142 is provided with an obstruction 143 which further insures that a male luer fitting will not be coupled to the proximal end of dilator 110. As shown in Figure 10, obstruction 143 protrudes into lumen 122 portion of dilator hub 120 starting at the proximal end 116 of hollow tube 115. In this fashion, a guide wire fed into distal opening 117 and advanced through hollow tube 115 into dilator hub 120 will not be obstructed. An obstruction having any configuration which allows a guide wire to be passed through the dilator hub from the distal direction without obstruction while denying entry to a tube or luer fitting from the proximal direction would achieve the benefits of this aspect of the present invention. Figure 11 is a proximal, end view of dilator 110 clearly illustrating the lumen 142 with obstructing member 143. Figure 12 is an end view of dilator 110 taken from the distal end showing distal hole 117 in tube 115.

The dilator hub of the present invention may be formed using well known injection molding processes with any bio-compatible materials such as the thermoplastics generally used in the medical field.

In contrast to conventionally designed dilators, the present invention therefore prevents the coupling of other medical devices such as IV lines and catheters to the improved dilators disclosed herein.

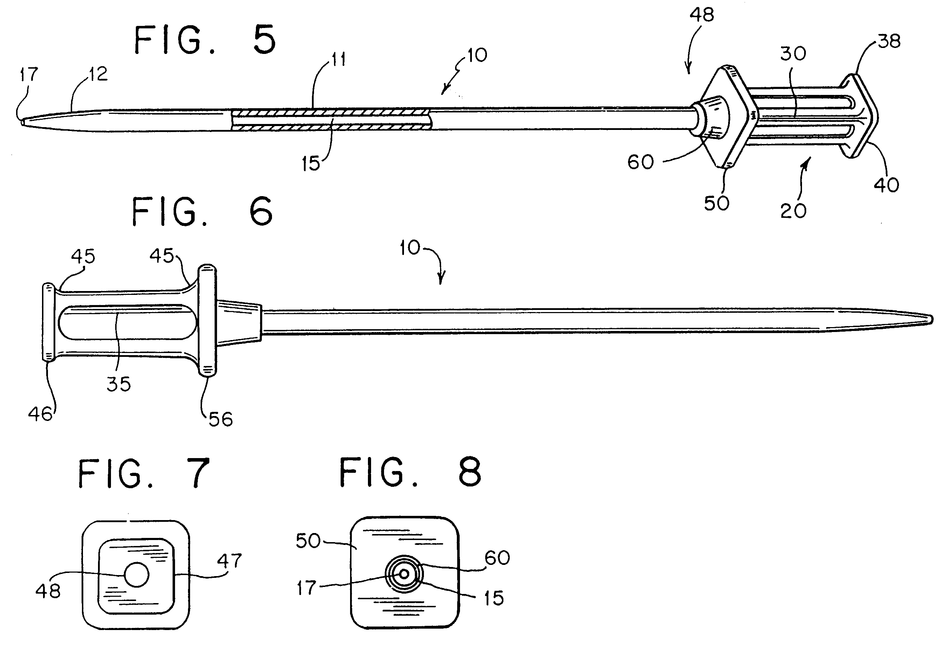

Figures 3 and 5-8 also illustrate another aspect of the present invention. In order to facilitate the comfortable and secure handling of the dilator 10 of the present invention, a gripping region 30 of this illustrated embodiment is positioned between a proximal flange 40 and a distal flange 50. Thus, the general shape of this dilator hub 20 conforms to the shape of the user's fingers providing a more comfortable feel than conventional dilators such as those shown in Figures 1 and 2. With reference to the side view of Figure 6, the rims of these flanges are advantageously formed with rounded edges 46, 56. Gripping region 30 advantageously meets both flanges with a smooth, gradually sloping contour 45 which provides greater comfort than the right angles of conventional dilator hubs.

While other configurations are within the scope of the present invention, Figures 7 and 8 illustrate the substantially square configuration of each of the flanges wherein substantially straight sides 48 meet in rounded corners 47.

According to this illustrated first embodiment of the present invention, a comfortable, secure grasp is provided by positioning the proximal flange 40 and distal flange 50 at the ends of the gripping region 30. For example, the distance between the respective flanges is preferably about 0.76 to 3.17 cm (0.3 to 1.25 inches) most preferably about 1.27 to 2.03 cm (0.5 to 0.8 inches) and is designed to be grasped between a thumb and second finger. Medical personnel may desire to hold the dilator with other fingers, such as the thumb and middle finger.

As best shown in Figure 6, the flanges of this illustrated first embodiment are provided with effective circumferences greater than that of the gripping region 30. Distal flange 50 advantageously provides an abutment for fingers when the dilator is being moved distally during an insertion procedure. Either flange can similarly assist in the exertion of a proximally-directed, pulling force during withdrawal of dilator 10 from a patient.

The gripping surface 30 is preferably provided with a roughened, textured surface designed to increase the friction of the gripping region. For example, if the dilator hub is formed of a molded thermoplastic, the mold may be provided with a slightly roughened surface to yield a dilator hub surface which will not readily become slick. Alternatively, the gripping surface may be slightly knurled. The illustrated gripping region 30 is also advantageously provided with four longitudinally extending slots 35 which extend substantially the entire length of the gripping region 30 and which facilitate the rotation of dilator 10 during a surgical procedure. Fewer or more longitudinal slots are also within the scope of the present invention.

A comparison of the embodiment of the present invention illustrated in Figure 4 with the previously known catheter hubs illustrated in Figures 1 and 2, indicates that the dilator hub of the present invention has a more ergonomically designed gripping region. The gripping region 30 has a substantially constant effective circumference between flange regions 40 and 50.

As used herein, the term "effective circumference" at any given point along the length of the dilator hub is meant to indicate a theoretical circumference calculated using a diameter equal to the longest, measurable distance passing through a central longitudinal axis of the dilator hub. Thus, the effective circumference of the proximal flange region 40 would be calculated with a diameter equal to the distance between two opposing corners 47 of the proximal flange region. The "effective circumference" of the proximal end of the dilator hub illustrated in Figure 1 would be calculated using a diameter equal to a distance between the edges of opposing ears 4. With this definition in mind, it will be appreciated that the dilator hub illustrated in Figure 1 has four different effective circumferences (excluding the collar adjacent to the semi-rigid tube) with sharp, right angles defining the borders of these four areas. The dilator hub illustrated in Figure 2 has five regions of different effective circumferences. These numerous changes in the effective circumference on previously known dilator hubs fail to provide a comfortable gripping region. In contrast, dilator hub 20 shown in Figure 4 has a gripping region 30 with a substantially constant effective circumference for at least one-half inch along the length of the dilator hub, more preferably for at least five eighths of an inch along that length. As shown in Figure 6, the gripping region 30 also slopes gradually from gripping region 30 to each of the flanged regions thereby providing a more comfortable feel than the right angles on conventionally known dilator hubs.

According to one alternative embodiment of the present invention illustrated in Figures 4 and 9-12, a dilator hub 120 is provided with an even smooth profile. As illustrated in the figures, the gripping section 130 of dilator 110 slopes very gradually and continuously to proximal flange section 140 and distal flange section 150. It will be appreciated that this embodiment of the present invention may be found even more comfortable by medical personnel.

While the illustrated embodiments of the present invention each comprise two flange regions positioned distally and proximally of the gripping region, a smooth, comfortable dilator hub having sufficient friction for gripping may be provided by including only one of these flanged regions. Thus, it will be appreciated that one aspect of the present invention provides a comfortable dilator hub having a gripping region with a generally smooth contour and at least one flanged region disposed adjacent the gripping region to further facilitate the application of a distally and/or proximally-directed pressure by medical personnel.

The dilator hub 120 of this embodiment is similarly provided with an attachment collar 160 to facilitate a secure bond between dilator hub 120 and hollow tube 115. The external surface of dilator hub 120 may also be provided with a non-slip texture as stated above with respect to the previously described embodiment.

## Claims

1. A dilator (10) for dilating a vessel prior to introduction of a catheter into the vessel, the dilator (10) comprising a tube (11) having a lumen (15), a proximal end and a distal end (12), said distal end (12) being adapted for insertion into a vessel and said proximal end being shaped to permit guiding means to pass through said lumen (15) characterized in that said tube (11) includes a means for obstructing (42/143) the proximal end of said lumen (15) to prevent luer connector means from being connected to said proximal end.

2. The dilator (10) of claim 1 wherein said proximal end comprises hub means (20) with a hub opening (42) connected to said proximal end of said tube (11), said hub opening (42) being adapted to prevent liner connector means from being connected to said proximal end of said dilator (10).

3. The dilator (10) of claim 2 wherein said hub opening (42) has a diameter of between about 0.05 cm (0.020 inches) to about 0.343 cm (0.135 inches).

4. The dilator (10) of claim 2 further comprising obstruction means (143) located in said hub lumen to prevent liner connector means from being connected to said hub means (20).

5. The dilator (10) of claims 2, 3 or 4 wherein said hub means (20) comprises a generally squared end (38).

6. The dilator (10) of claims 2, 3 or 4 wherein said hub means (20) comprises a flanged end (40).

## Patentansprüche

1. Dilatator (10) zum Dilatieren eines Gefäßes vor der Einführung eines Katheters in das Gefäß, wobei der Dilatator (10) ein Rohr (11) mit einem Lumen (15), einem proximalen Ende und einem distalen Ende (12) aufweist, welches distale Ende (12) zum Einsetzen in ein Gefäß geeignet ist, und welches proximale Ende geformt ist, um einem Führungsmittel zu gestatten, durch das Lumen (15) hindurchzugehen, dadurch gekennzeichnet, daß das Rohr (11) ein Mittel zum Blockieren (42/143) des proximalen Endes des Lumens (15) enthält, um Luer-Verbindermittel daran zu hindern, mit dem proximalen Ende verbunden zu werden.

2. Dilatator (10) nach Anspruch 1, wobei das proximale Ende ein Nabenmittel (20) mit einer Nabenöffnung (42) aufweist, die mit dem proximalen Ende des Rohrs (11) verbunden ist, welche Nabenöffnung (42) geeignet ist, Luer-Verbindermittel daran zu hindern, mit dem proximalen Ende des Dilatators (10) verbunden zu werden.

3. Dilatator (10) nach Anspruch 2, wobei die Nabenöffnung (42) einen Durchmesser zwischen etwa 0,05 cm (0,020 Zoll) und etwa 0,343 cm (0,135 Zoll) aufweist.

4. Dilatator (10) nach Anspruch 2, welcher ferner ein Blockierungsmittel (143) aufweist, das im Nabenlumen angeordnet ist, um Luer-Verbindermittel daran zu hindern, mit dem Nabenmittel (20) verbunden zu werden.

5. Dilatator (10) nach Anspruch 2, 3 oder 4, wobei das Nabenmittel (20) ein allgemein rechteckig bearbeitetes Ende (38) aufweist.

6. Dilatator (10) nach Anspruch 2, 3 oder 4, wobei das Nabenmittel (20) ein mit einem Flansch versehenes Ende (40) aufweist.

## Revendications

1. Dilatateur (10) destiné à dilater un vaisseau avant l'introduction d'un cathéter dans le vaisseau, le dilatateur (10) comprenant un tube (11) ayant une lumière (15), une extrémité proximale et une extrémité distale (12), ladite extrémité distale (12) étant adaptée pour être insérée dans un vaisseau et ladite extrémité proximale étant conformée pour permettre à des moyens de guidage de passer à travers ladite lumière (15), caractérisé en ce que ledit tube (11) comprend un moyen (42/143) pour obstruer l'extrémité proximale de ladite lumière (15) pour empêcher de raccorder des moyens de raccord de Luer à ladite extrémité proximale.

2. Dilatateur (10) selon la revendication 1, dans lequel ladite extrémité proximale comprend un moyen formant embout (20) possédant une ouverture d'embout (42) reliée à ladite extrémité proximale dudit tube (11), ladite ouverture d'embout (42) étant adaptée pour empêcher de raccorder le moyen formant raccord de Luer à ladite extrémité proximale dudit dilatateur (10).

3. Dilatateur (10) selon la revendication 2, dans lequel ladite ouverture d'embout (42) a un diamètre d'entre environ 0,05 cm (0,020 pouce) et environ 0,343 cm (0,135 pouce).

4. Dilatateur (10) selon la revendication 2, comprenant en outre des moyens d'obstruction (143) logés dans ladite lumière d'embout pour empêcher de raccorder les moyens formant raccord de Luer auxdits moyens formant embout (20).

5. Dilatateur (10) selon les revendications 2, 3 ou 4, dans lequel lesdits moyens formant embout (20) comprennent une extrémité (38) sensiblement carrée.

6. Dilatateur (10) selon les revendications 2, 3 ou 4, dans lequel lesdits moyens formant embout (20) comprennent une extrémité à collerette (40).
